# EUROPEAN PATENT SPECIFICATION

(11) **EP 1 349 539 B1**
(45) Date of publication and mention of the grant of the patent: **13.10.2004**
(21) Application number: 02748997.0
(22) Date of filing: 08.07.2002
(51) Int. Cl.: A61K 9/00, A61L 27/54, A61K 31/616

(54) **BIOCOMPATIBLE COMPOSITE**
BIOKOMPATIBLES VERBUNDMATERIAL
COMPOSITE BIOCOMPATIBLE

(30) Priority: 11.07.2001 GB 0116920
(43) Date of publication of application: 08.10.2003
(73) Proprietor: VASCUTEK LIMITED, Renfrewshire, Scotland PA4 9RR (GB)
(72) Inventor: KELSO, Karen, Anne, Kilmarnock KA3 6BJ (GB); MAINI, Roshan, Bridge of Weir, PA11 3DL (GB)
(74) Representative: Ouzman, Beverley Nicola Claire
(86) International application number: PCT/GB2002/003108
(87) International publication number: WO 2003/005990

(56) References cited:
- EP-A- 0 537 006
- EP-A- 0 680 753
- WO-A-94/15588
- DATABASE CA [Online] CHEMICAL ABSTRACTS SERVICE, COLUMBUS, OHIO, US; HALL, JOHN D. ET AL: "Effect of controlled local acetylsalicylic acid release on in vitro platelet adhesion to vascular grafts" retrieved from STN Database accession no. 121:195386 XP002213702 & JOURNAL OF BIOMATERIALS APPLICATIONS ( 1994 ), 8(4), 361-84 , XP008008305

## Description

The present invention provides a novel and improved delivery system, and a method for the preparation thereof.

Vascular grafts are often unsuccessful due to complications. In particular restenosis, leading to intimal hyperplasia (IH) is known to contribute to early graft failure. Restenosis occurs due to the migration of vascular smooth muscle cells (VSMC) from the media layer to the intima layer where the cells proliferate and overproduce extracellular matrix. Blood vessels affected by restenosis are constricted or narrowed due to the uncontrolled growth of smooth muscle cells (SMC) with extracellular matrix deposition.

Intimal hyperplasia involves the abnormal migration and proliferation of medial smooth muscle cells to the intima layer causing narrowing of vessel lumen which can lead to thrombosis. If any of the endothelial layer of a vessel is exposed, platelet activation is initiated causing platelet derived growth factor (PDGF) release.

Smooth muscle cell migration and proliferation is induced by binding of SMCs to high affinity, up-regulated receptors in response to an injury. VSMC play a contributory role in intimal hyperplasia. Growth factors and mitogens from denudation of the endothelial layer cause activation of medial vascular smooth muscle cells (VSMC) and migration of VSMC from medial to intima layers changing the genetic phenotype from contractile to synthesis function. A large quantity of extracellular matrix is generated to provide support for the increased number of cells. VSMC are thus proliferated in the intima layer. Leucocytes also contribute to intimal hyperplasia. Vascular permeability and up-regulation of adhesion molecules is increased causing increased adhesiveness of leucocytes to vessel walls and increased infiltration of the tissue by the leucocytes. Leucocytes release inflammatory mediators, attracting more leucocytes to the site of the injury. Thus the injury is perpetuated by degrading key components of extracellular matrix.

There is clearly a need for a means of controlling the complications described above. Dexamethasone (Dex) is a synthetic glucocorticoid important in modulation and suppression of immune function. Aspirin (acetylsalicylic acid) is commonly used in anti-platelet therapy to prevent graft thrombosis and to suppress the development of intimal hyperplasia. Glyceryl Trinitrate (GTN) is an organic nitrate which is a muscle relaxant for vascular smooth muscles. It is commonly used to prevent coronary vasospasm associated with thrombolysis-induced platelet activation.

The controlled release of an active ingredient over a sustained period of time can be achieved using a polymer/active ingredient composite. Generally a polymer is combined with the chosen active ingredient, and controlled release of the active ingredient is due to slow release of the active ingredient from a biostable polymer or to the biodegradation of a biodegradable polymer. The active ingredient will usually be a pharmaceutical, but other active ingredients may also be contemplated for non-medical applications. Such composites rely upon the intimate admixture of the ingredients, with the active ingredient being evenly dispersed throughout the polymer to ensure that the rate of release of the active ingredient remains uniform. Consequently the active ingredient must be in a form able to achieve even dispersion throughout the polymer.

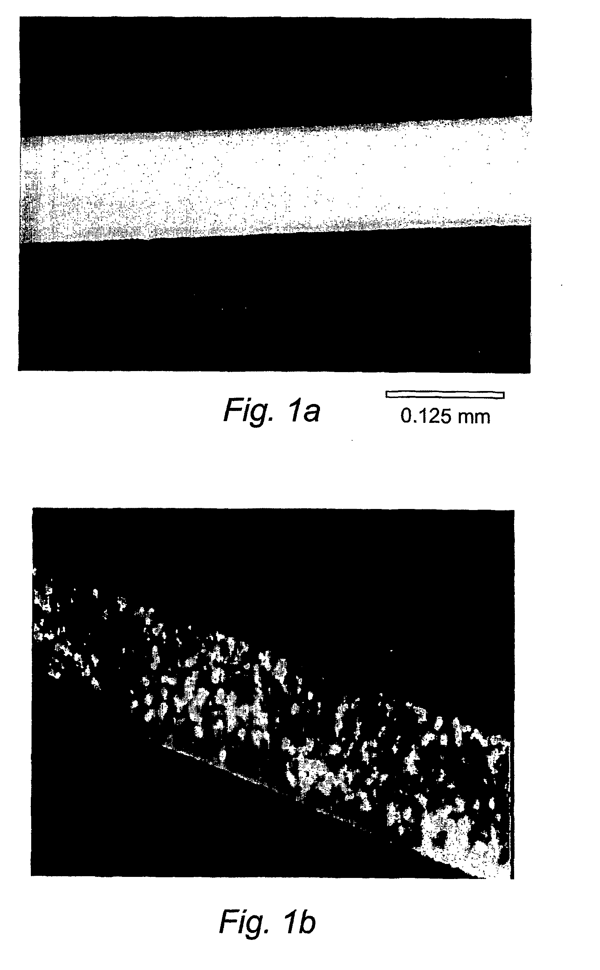

A particular problem occurs when it is desired that poorly soluble active ingredients, for example aspirin, are integrated into a polymer/active ingredient composite. There is a tendency for such active ingredients to crystallise in large clumps (Fig. 1b), rather than achieving a homogeneous mix (Fig. 1a).

Whilst the active ingredient can be simply loaded into a pre-cured polymer, the level of controlled release achievable is often limited, since the amount of active ingredient available for release depends upon the loading achieved.

Ultrasound is known to be useful for mixing components together, particularly to produce homogeneous suspensions. Ultrasonication has been employed in the production of drug compositions, generally for the production of drug/phospholipid complexes.

Thus, EP-A-0,161,445 describes the use of ultrasound in the formation of a water-insoluble drug/phospholipid liposome, the liposome itself having the advantage of being water soluble.

GB 937,303 also describes the use of ultrasound in the preparation of a homogenous suspension of a medicament in the form of a very fine powder. The powder is produced by precipitation of the medicament and ultrasound is employed doing precipitation to ensure that the particles so formed are very fine.

Neither of the above publications refers to a polymer/active ingredient composite.

In US 4,898,734 microcapsules or microspheres containing a medicament or other active ingredient are embedded in a polymer. The spheres are homogenously dispersed in the polymer and are compelled to release their contents upon exposure to temperature, light or ultrasound.

In WO-A-00/69942 a micelle or liposome formed from a hydrophobic/hydrophilic block polymer is described. The micelle has a stabilised core, and drugs can be incorporated into the stable inner core of the micelles. When subjected to ultrasound the drugs are released from the core, but this is reversible so that when the ultrasound is switched off, the remaining drugs are re-encapsulated. By pulsing the ultrasound, controllable release of the drugs can be achieved.

In both of the above publications, ultrasound is used to achieve release of the active substance.

US 5,620,697 describes a method of preparing pharmaceutical compositions, using ultrasound. A biodegradable polymer matrix and at least one pharmaceutical mixed or dissolved in the matrix are subjected to ultrasound so that the mixture of polymer and pharmaceutical substance is at least partially melted. Thus ultrasound is used to plasticise and mould the drug delivery system.

We have now found that ultrasound can be used in a more efficient process to promote even dispersion of an active ingredient in a polymer, even where the active ingredient is poorly soluble and has a tendency to crystallise upon exposure to the polymer. Further, we have found that ultrasound can be used to promote the loading of a pre-cured polymer with an active ingredient and also to promote uptake of the active ingredient into the cured polymer.

Thus, the present invention provides a method of producing a biocompatible composite, said method comprising the step of combining a polymer and an active ingredient wherein ultrasound having a frequency of 20 to 90 kHz is used during the combination of said polymer and said active ingredient.

Desirably the polymer/active ingredient combination is exposed to ultrasound for a time sufficient to ensure that an even distribution of active ingredient throughout the polymer matrix is achieved.

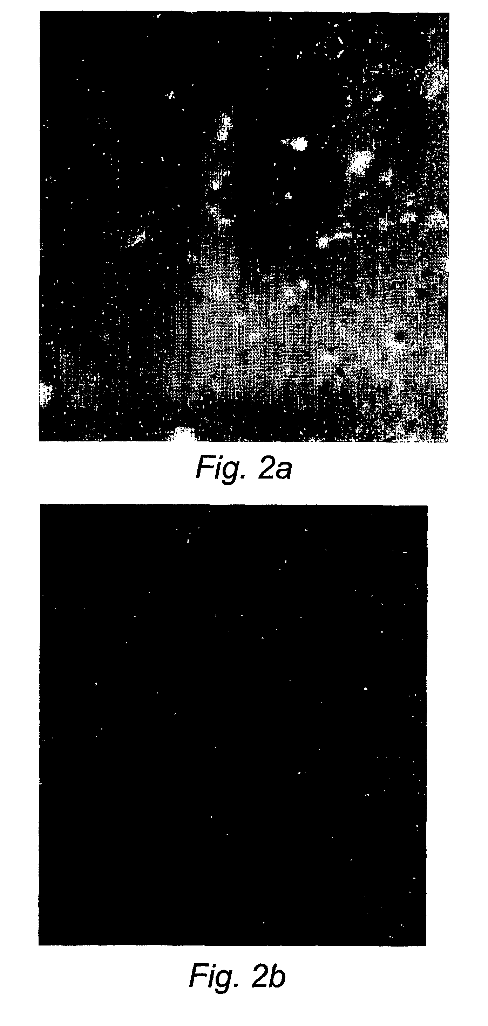

In one embodiment, the polymer is in solution or suspension and cure of the polymer is subsequent to combination with the active ingredient and the ultrasonication step (Fig. 2b).

In an alternative embodiment, the polymer is cured prior to exposure to the active ingredient and the ultrasonication step (Fig. 1a).

The composite produced according to the above method forms a further aspect of the present invention.

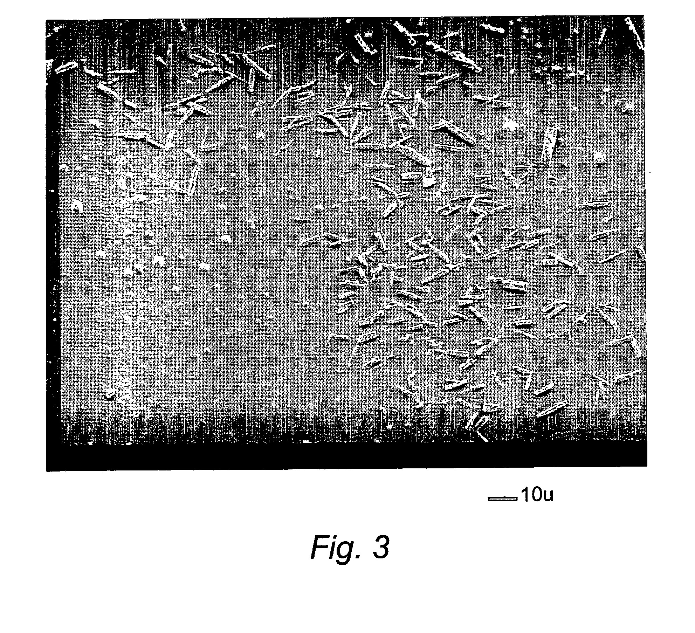

As mentioned above, the present invention is particularly of utility for poorly soluble active ingredients, such as Aspirin (acetylsalicyclate), which demonstrate a tendency to crystallise on the polymer. Attempts to combine aspirin dissolved in chloroform with a silicone polymer solution by stirring merely led to large granular lumps of aspirin forming on the cured polymer (see Fig. 2a). Alternative attempts, where the polymer had been cured and then dipped into the stirred aspirin solution, resulted in no penetration into the polymer by the aspirin being achieved; instead aspirin crystallised on the surface of the polymer (see Figure 3).

The polymers for use in the present invention may be any pharmaceutically acceptable polymer. The polymer may be biostable or may be biodegradable. Exemplary polymers include (but are not limited to) silicones, silicone-based polymers (for example fluorosilicone), polyesters, polyurethanes, polyorthoesters and poly-α-hydroxy acids such as polyglycolide (PGA), polylactides (PLA), polyhydroxybutyrate (PHB) and co-polymers thereof (for example PHB/polyhydroxyvalerate copolymer). Silicone, fluorosilicone and polyurethane are preferred.

The active ingredient may be any active ingredient compatible with ultrasonic processing. Examples include antibiotics, polypeptides and steroidal hormones such as dexamethasone. Anti-platelet, anti-proliferative and anti-microbial agents are also suitable. The invention is of particular utility for poorly soluble active ingredients which would otherwise have a tendency to form crystals. Aspirin is an especially preferred active ingredient, but other active ingredients of importance include NO donors, such as glycerol trinitrate. For aspirin an exemplary solution range is 100mg to 500mg in 10ml chloroform. We have found that the solvent evaporates from the composite and is not retained therein. Two or more active ingredients may be combined with the polymer.

The frequency of ultrasound used may be in the range of 20-90kHz, for example 30-50kHz, especially 40kHz.

In the present invention, the active ingredient and polymer may each be dissolved in separate solvents or dispersed in appropriate liquid carriers and then combined together. Ultrasound may then be used to promote even admixture prior to the polymer being cured. We have found that the composite appears to have some protecting effect on the active ingredient incorporated in this way. Specifically, polymer curing at temperatures of 120ºC for 45 minutes has been acceptable for aspirin, the melting point of which is 118ºC.

Optionally, the solution/suspension of the active ingredient may be subjected to ultrasound prior to admixture with the polymer. Likewise the solution/suspension of polymer may undergo ultrasonication before admixture with the active ingredient. We have found that such additional preliminary ultrasonication steps enhance the final product, and particularly good results may be achieved when both active ingredient and polymer are separately ultrasonicated, then combined and the admixture ultrasonicated again.

Where the polymer is combined with the active ingredient in solution, that is to say prior to curing, it is possible to form a coating of the composite on a substrate. This is of particular interest where the substrate is intended for (temporary or permanent) implantation into a patient and the active ingredient held within the composite reduces immune rejection, embolism formation, scar formation, infection, or promotes healing. The substrate may simply be dipped into the admixture of polymer and active ingredient either during or subsequent to ultrasonication.
The substrate may be formed of any material suitable to be coated with the polymer. Typically, the substrate will be a thermoplastic elastomer, but other specific examples include nitinol, polyester and ePTFE.

The coated substrate may be used for any designated purpose, depending upon the active ingredient incorporated in the composite, but mention may be made of catheters, stents, embolism filters and heart valve leaflets.

Alternatively the polymer may be cured in the required form (for example as a sheet, typically a few millimetres thick) and dipped into a solution or suspension of the active ingredient which is then ultrasonicated. Typically, a timescale of 30 minutes to 2 hours, for example 1 hour, is sufficient to allow total loading of a 3 mm thick polymer film. Alternative thicknesses of polymer sheeting or indeed alternative polymer shapes (for example tubes or rods) may also be employed.

Surprisingly, we have found that ultrasound promotes the absorption of the active ingredient into the interstices of the cured polymer, as well as achieving a more even coating thereon.
For certain polymer types we have found that a heating step is beneficial in retaining the drug within the polymer. For example, we have found that repeated (e.g. 3 or 4) short periods (e.g. of 10 minutes) of heating to a temperature of 80 to 110ºC assists retention of aspirin in silicone and fluorosilicone polymers, but is not required for polyurethane polymers.

In composite polymers of the type of interest here, incorporation of the active ingredient leads to a reduction in the tensile strength of the polymer. High loading of active ingredient (either by use of a concentrated solution or a long period of incorporation in the presence of ultrasound) may lead to unacceptably low tensile strength characteristics. However, the more homogeneous distribution of the active ingredient in the present invention reduces the incidence of areas in the composite where tensile failure is likely due to the presence of large deposits of active ingredients in a single location.

In summary, the use of ultrasound as described above enables a homogenous distribution of the active ingredient within the polymer, and achieves a minimal particle size for the active ingredient.
The method of the present invention minimises the required preparation times and is useful for both pre-cure and post-cure loading of the polymer.

According to a further aspect of the present invention there is provided a composite obtainable from the method described above, comprising a polymer and an active ingredient, said active ingredient being evenly dispersed in said polymer.

According to a further aspect of the present invention there is provided a vascular graft comprising a composite as described above.

The present invention will now be further described with reference to the examples and figures in which:
Figure 1 shows a cross-section through silicone sheet post-loaded with acetylsalicylate (aspirin) using ultrasound (a) and without ultrasound (b).
Figure 2 shows silicone sheet pre-loaded with acetylsalicylate (aspirin) without ultrasound (a) and with ultrasound (b).
Figure 3 shows a magnified image of cured silicone sheet soaked in aspirin solution for one hour. Aspirin has formed characteristic crystalline structures on and within the silicone in the absence of ultrasound. The aspirin distribution is highly disorganised with non-uniform topography.

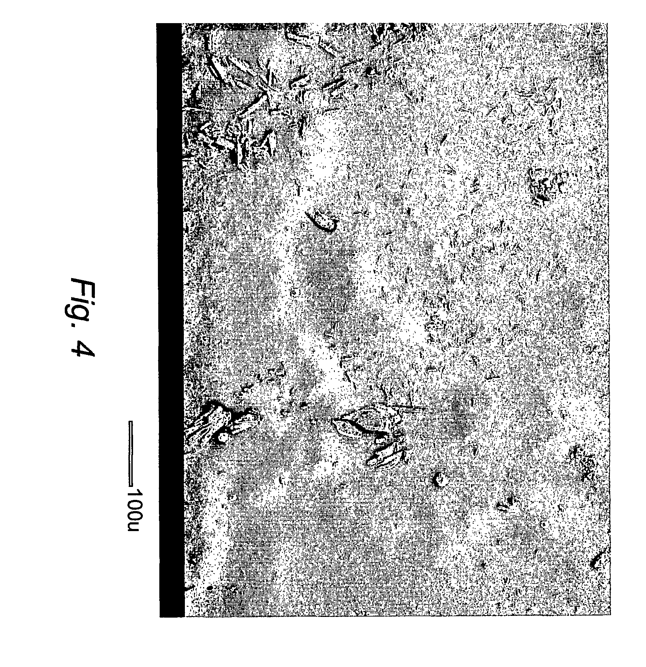

Figure 4 shows a magnified image of cured silicone sheet post-loaded with aspirin. The cured sheet was soaked in aspirin solution for one hour and ultrasonicated. No crystalline structures are seen, aspirin distribution is highly organised and uniform with minimal particle size distribution.

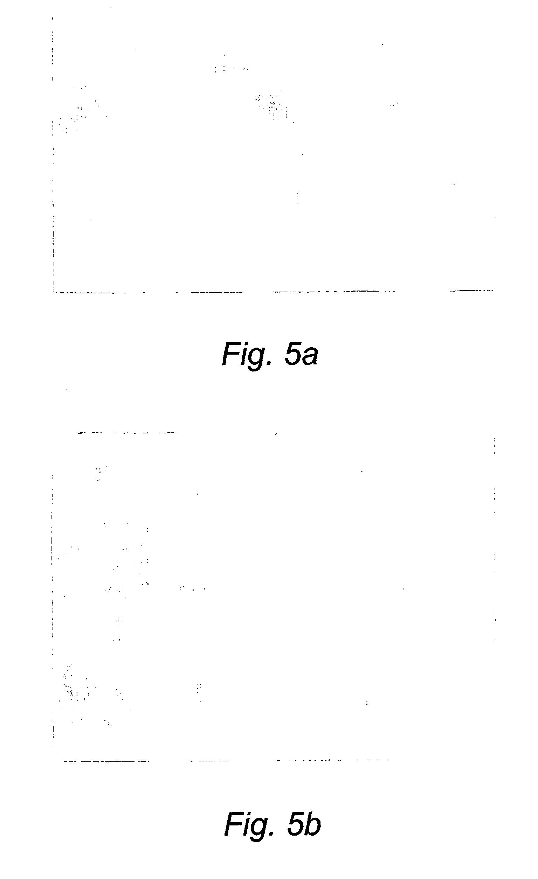

Figures 5a and b show images taken by light microscope of silicone sheet post-loaded with aspirin without ultrasonication. Darker patches show areas of non-penetration by aspirin in the absence of ultrasound.

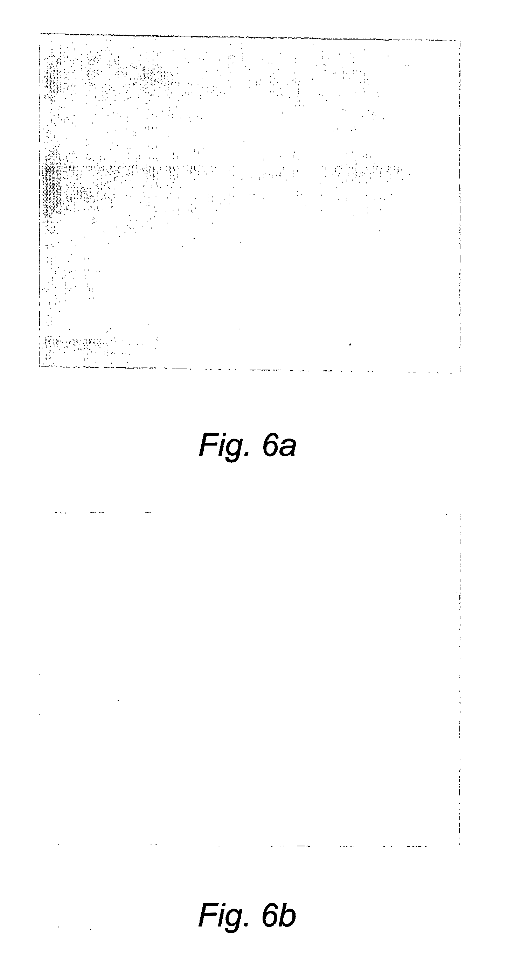

Figures 6a and b show images taken by light microscope of silicone sheet post-loaded with aspirin with ultrasound. No dark patches or areas of non-penetration of aspirin are seen. There is uniform distribution.

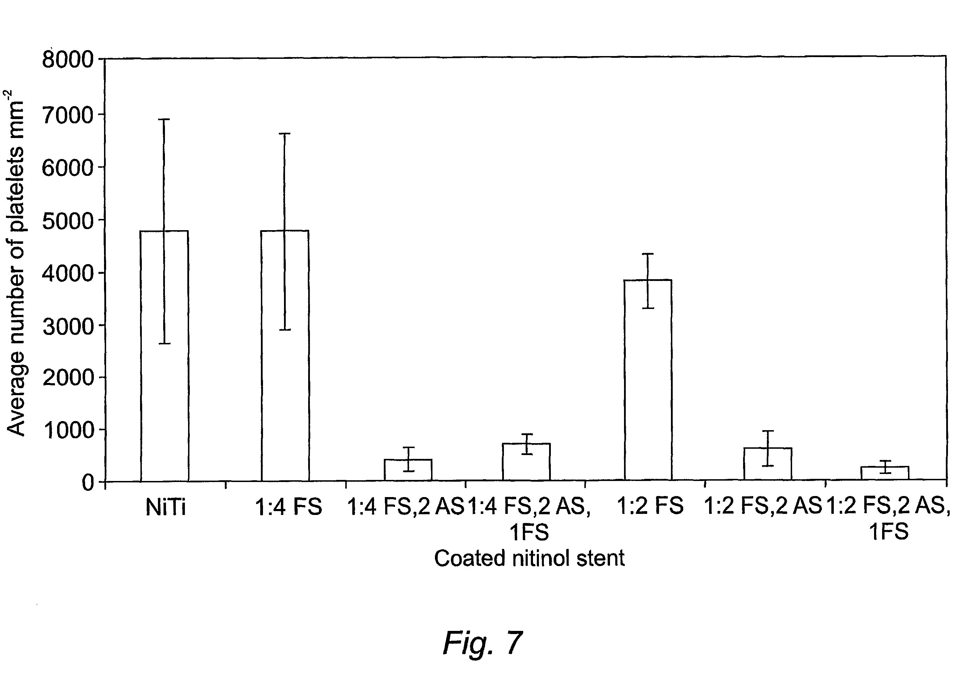

Figure 7 shows the average number of platelets per square millimetre of stents coated with various coatings.

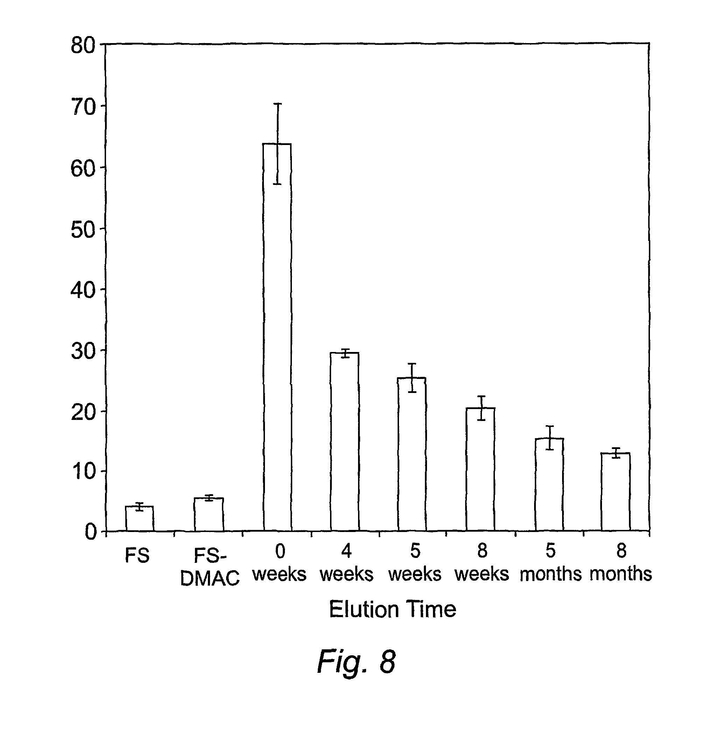

Figure 8 shows the amounts of aspirin in eluted fluorosilicone films. The different films were eluted for different amounts of time.

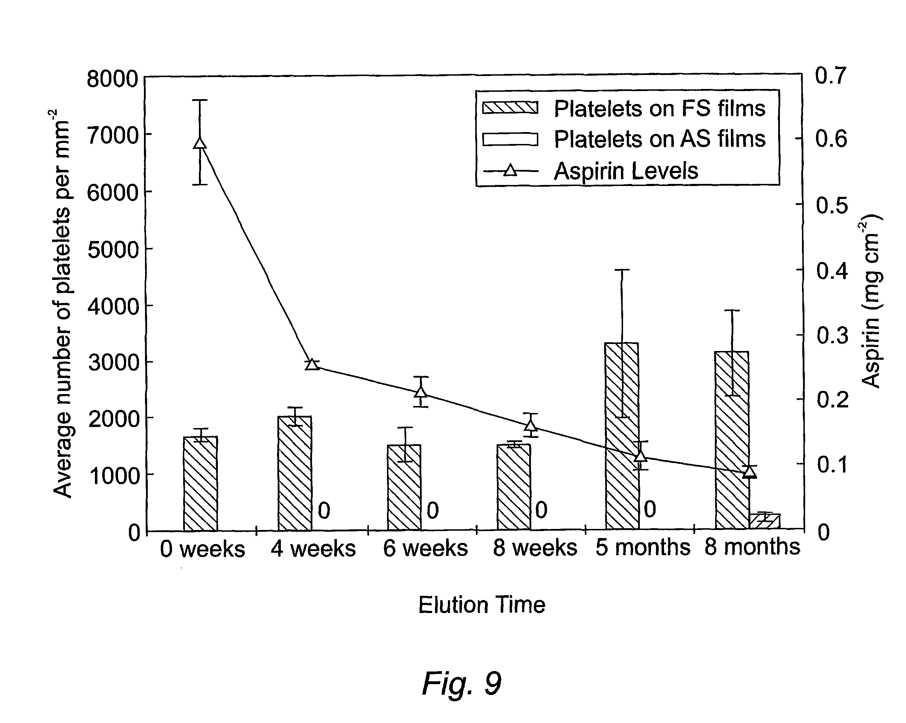

Figure 9 compares the aspirin levels and the average number of platelets on Fluorosilicone and aspirin films. The different films were eluted for different amounts of time.

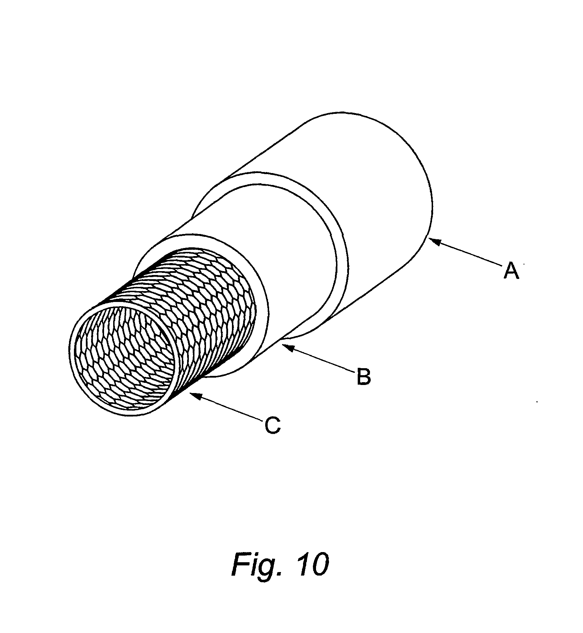

Figure 10 shows a partial cross sectional view of a blood vessel wherein layer A shows the tunica layer made up of connective tissue, layer B shows the medial layer made up of smooth muscle cells and layer C shows the intima layer made up of endothelial cells.

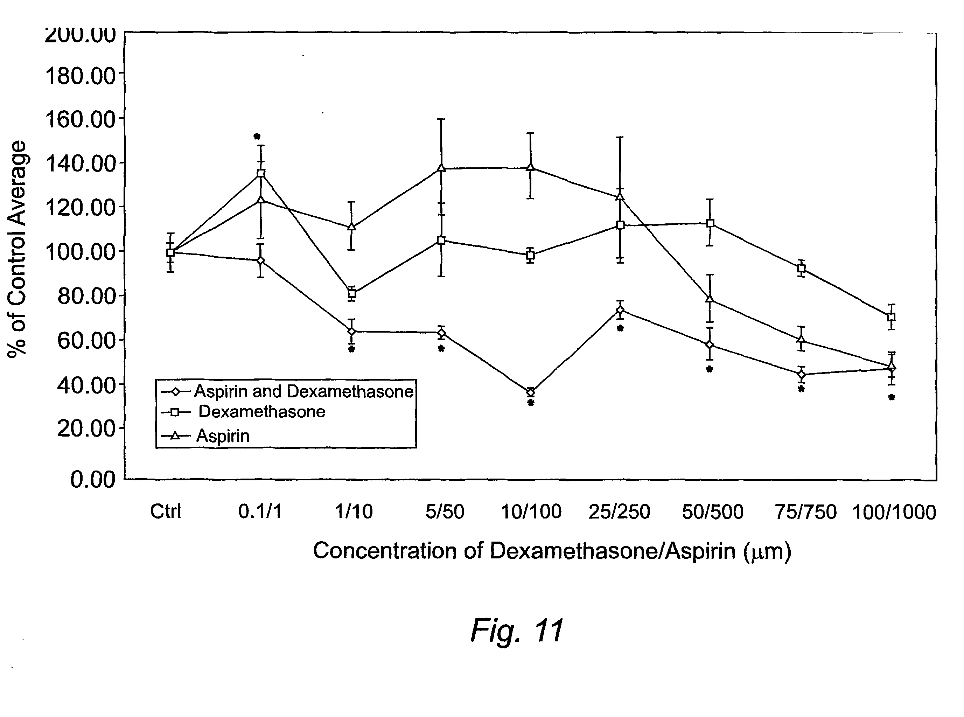

Figure 11 shows the results of an MTT test comparing the concentration of aspirin, dexamethasone and a combination of aspirin and dexamethasone on a seeded well plate, as a function of a percentage of the control average, the control being ethanol.

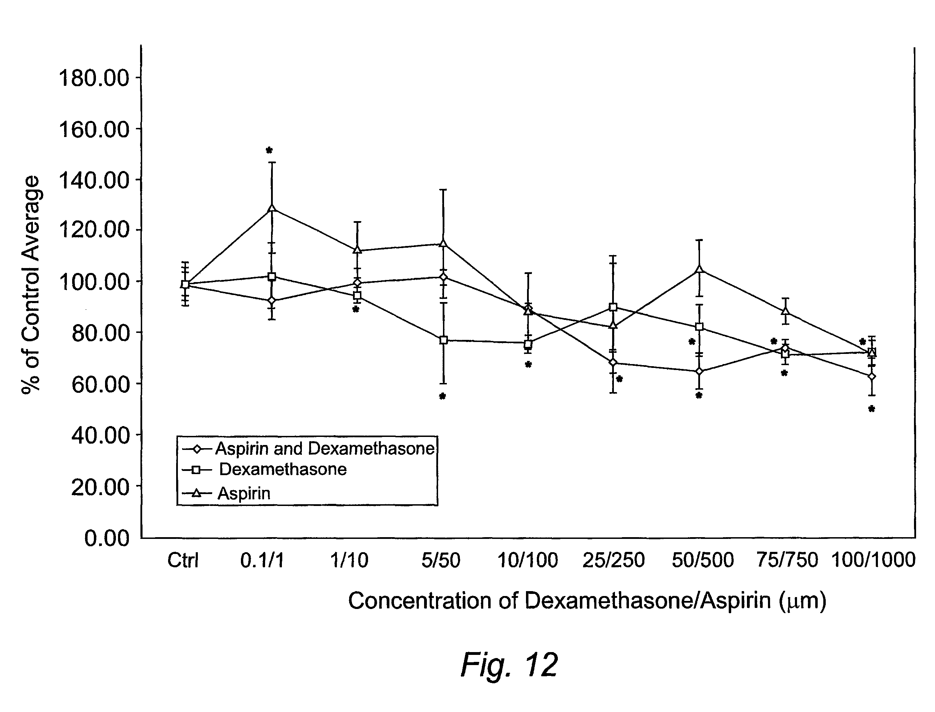

Figure 12 shows the results of an Neutral Red assay (NRA) test comparing the concentrations of aspirin, dexamethasone and a combination of aspirin and dexamethasone on a seeded well plate, as a function of a percentage of the control average, the control being ethanol.

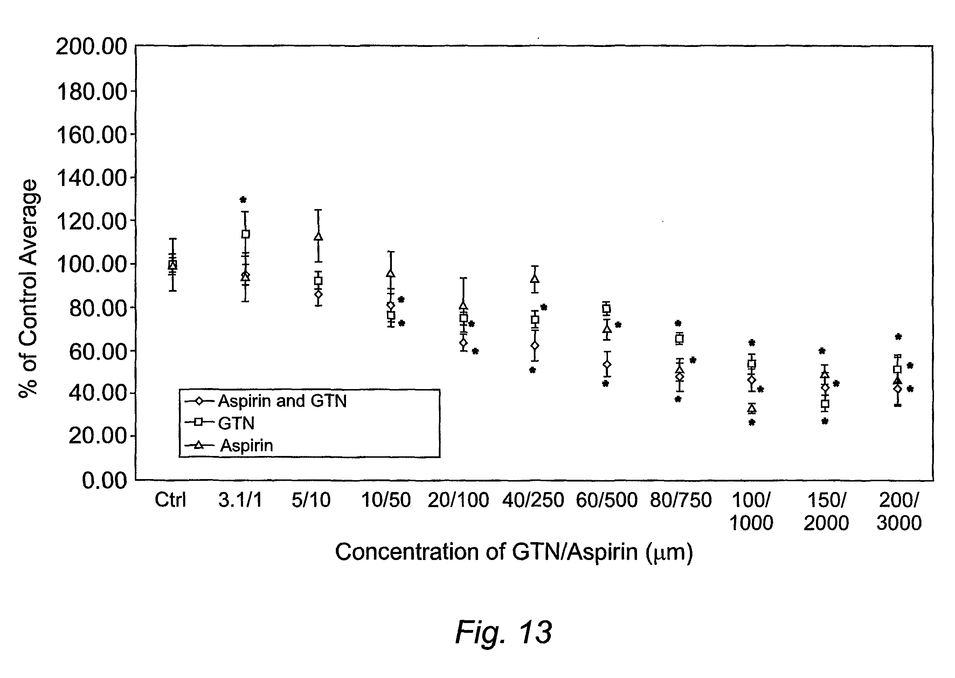

Figure 13 shows the results of a Neutral Red assay (NRA) test comparing the concentration of aspirin, GTN and a combination of aspirin and GTN on a seeded well plate, as a function of a percentage of the control average, the control being F12-K.

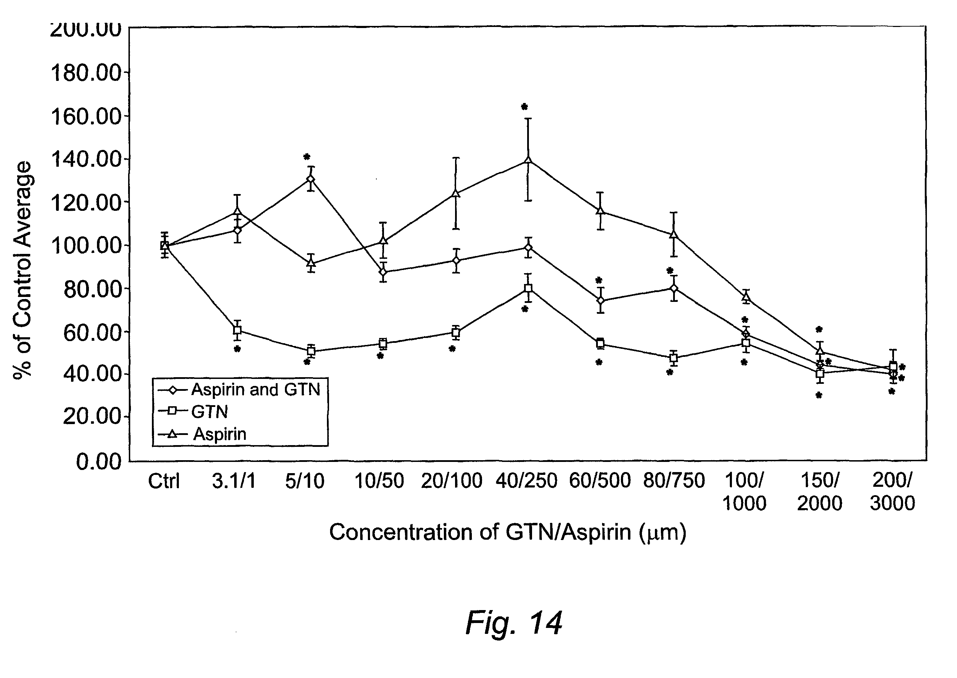

Figure 14 shows the results of an MTT test comparing the concentration of aspirin, GTN and a combination of aspirin and GTN on a seeded well plate, as a function of a percentage of the control average, the control being F12-K.
Figure 15 shows the Confocal Laser Scanning Microscope (CLSM) images of cellular samples (FFC bone cells). Wherein Figure 15a shows the CLSM image of a positive control which contains a primary and secondary antibody, Figure 15b shows the CLSM image a negative control which contains a secondary antibody and Figure 15c shows the CLSM image of a control control which does not contain primary or secondary antibody. The fluorescence of the samples equates to collagen production in FFCs (bone cells).
Figure 16 shows the Confocal Laser Scanning Microscope (CLSM) images of samples after fixation and staining with acridine orange wherein 16a shows the CLSM image of a control glass coverslip which shows red fluorescence, 16b shows the CLSM image a control plastic petri dish which shows green fluorescence, 16c shows the CLSM image of a F1Si+GTN coated membrane which shows green fluorescence and 16d shows the CLSM image of a F1Si coated membrane which shows red fluorescence.
   Green fluorescence indicates binding of acridine orange to DNA and red fluorescence indicates binding of acridine orange to RNA, on a glass coverslip.
Figure 17 shows the Confocal Laser Scanning Microscope (CLSM) images of F1Si membrane on a glass coverslip after fixation and staining with acridine orange wherein 17a shows cells on the surface of the membrane, with red fluorescence, 17b shows cells on the glass coverslip to the edge of the membrane with red fluorescence, 17c shows cells on the glass coverslip with an attachment to the membrane and shows green fluorescence.

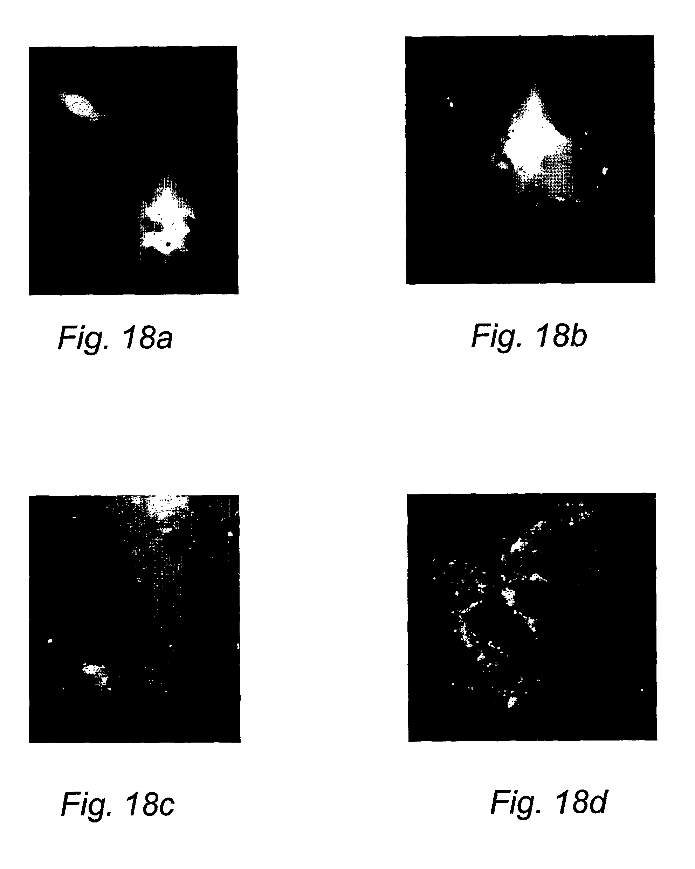

Figure 18 shows Confocal Laser Scanning Microscope (CLSM) images from viability studies wherein 18a shows a Confocal Laser Scanning Microscope (CLSM) image of cells on a control plastic petri dish after fixation and staining with acridine orange. The image shows green fluorescence, 18b shows a Confocal Laser Scanning Microscope (CLSH) image of cells on a control glass coverslip. This image shows green fluorescence. 18c shows cells on a F1Si only coated membrane. This image shows green fluorescence. 18d shows cells on a F1Si+GTN coated membrane.

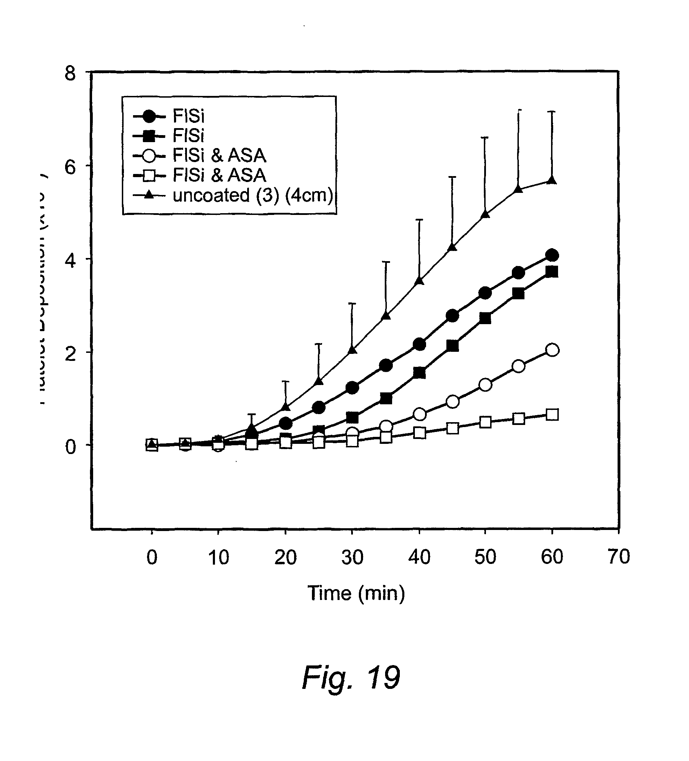

Figure 19 shows the effects of stent coating on thrombus formation.

### EXAMPLES 1 TO 3 : PRE-CURE LOADING OF ACETYLSALICYLIC ACID

### Example 1

Dissolve 20ml of fluorosilicone dispersion in 80ml of perchloroethylene:butylacetate (95:5 %v/v).
Place on an orbital shaker at 200 rpm 50°C for 1 hour. Remove and ultrasonicate in an ultrasonic bath for 30 minutes.

Dissolve 1g of acetylsalicylic acid in 2 ml of dimethylacetamide. Place immediately into an ultrasonic bath for 60 minutes.

Add the acetylsalicylic acid solution to 80 ml of fluorosilicone dispersion. Ultrasonicate for 60 minutes.

Cast films of fluorosilicone dispersion on glass slides. Dry for 1 hour. Cure for 45 minutes at 120°C.

### Example 2

Dissolve 20ml of fluorosilicone dispersion in 60ml of perchloroethylene:butylacetate (95:5 % v/v).
Place on an orbital shaker at 200 rpm 50°C for 1 hour. Remove and ultrasonicate in an ultrasonic bath for 30 minutes.

Dissolve 1g of acetylsalicylic acid in 2 ml of dimethylacetamide. Place immediately into an ultrasonic bath for 60 minutes.

Add the acetylsalicylic acid solution to 80 ml of fluorosilicone dispersion. Ultrasonicate for 60 minutes.

Cast films of fluorosilicone dispersion on glass slides. Dry for 1 hour. Cure for 45 minutes at 120°C.

### Example 3

Dissolve 30ml of fluorosilicone dispersion in 60ml of perchloroethylene:butylacetate (95:5 % v/v).
Place on an orbital shaker at 200 rpm 50°C for 1 hour. Remove and ultrasonicate in an ultrasonic bath for 30 minutes.

Dissolve 1g of acetylsalicylic acid in 2 ml of dimethylacetamide. Place immediately into an ultrasonic bath for 60 minutes.

Add the acetylsalicylic acid solution to 80 ml of fluorosilicone dispersion. Ultrasonicate for 60 minutes.

Cast films of fluorosilicone dispersion on glass slides. Dry for 1 hour. Cure for 45 minutes at 120°C.

The above examples were repeated using silicone and polyurethane as polymer.

### EXAMPLES 4 TO 8 : POST-CURE LOADING OF ACETYLSALICYLIC ACID

### Example 4

Dissolve 500 mg of acetylsalicylic acid in 10 ml of chloroform. Ultrasonicate for 60 minutes. Place clean silicone sheeting into the chloroform solution. Ultrasonicate for 60 minutes. Remove the silicone sheeting and dry on high speed 3D rotisserie for 1 hour.

Place dry silicone sheeting in an oven at 115 to 120°C for 10 minutes, remove and leave at room temperature for 10 minutes. Repeat this process a further 3 times.

### Example 5

Dissolve 500 mg of acetylsalicylic acid in 10 ml of chloroform. Ultrasonicate for 60 minutes. Place clean silicone sheeting into the chloroform solution. Ultrasonicate for 60 minutes. Remove the silicone sheeting and dry on high speed 3D rotisserie for 1 hour.

Place dry silicone sheeting in an oven at 115 to 120°C for 10 minutes, remove and leave at room temperature for 10 minutes. Repeat this process a further 2 times.

### Example 6

Dissolve 20ml of fluorosilicone dispersion in 80ml of perchloroethylene:butylacetate (95:5 % v/v).
Place on an orbital shaker at 200 rpm 50°C for 1 hour. Remove and ultrasonicate in an ultrasonic bath for 30 minutes. Cast films of fluorosilicone dispersion on glass slides. Dry for 1 hour. Cure for 45 minutes at 120°C.

Dissolve 500 mg of acetylsalicylic acid in 10 ml of chloroform. Ultrasonicate for 60 minutes. Place cured fluorosilicone into the chloroform solution. Ultrasonicate for 60 minutes. Remove the fluorosilicone and dry on high speed 3D rotisserie for 1 hour.

Place fluorosilicone in an oven at 115 to 120°C for 10 minutes, remove and leave at room temperature for 10 minutes. Repeat this process a further 3 times.

### Example 7

Dissolve 20ml of fluorosilicone dispersion in 60ml of perchloroethylene:butylacetate (95:5 % v/v).
Place on an orbital shaker at 200 rpm 50°C for 1 hour. Remove and ultrasonicate in an ultrasonic bath for 30 minutes. Cast films of fluorosilicone dispersion on glass slides. Dry for 1 hour. Cure for 45 minutes at 120°C.

Dissolve 500 mg of acetylsalicylic acid in 10 ml of chloroform. Ultrasonicate for 60 minutes. Place cured fluorosilicone into the chloroform solution. Ultrasonicate for 60 minutes. Remove the fluorosilicone and dry on high speed 3D rotisserie for 1 hour.

Place fluorosilicone in an oven at 115 to 120°C for 10 minutes, remove and leave at room temperature for 10 minutes. Repeat this process a further 3 times.

### Example 8

Dissolve 30ml of fluorosilicone dispersion in 60ml of perchloroethylene:butylacetate (95:5 % v/v).
Place on an orbital shaker at 200 rpm 50°C for 1 hour. Remove and ultrasonicate in an ultrasonic bath for 30 minutes. Cast films of fluorosilicone dispersion on glass slides. Dry for 1 hour. Cure for 45 minutes at 120°C.

Dissolve 500 mg of acetylsalicylic acid in 10 ml of chloroform. Ultrasonicate for 60 minutes. Place cured fluorosilicone into the chloroform solution. Ultrasonicate for 60 minutes. Remove the fluorosilicone and dry on high speed 3D rotisserie for 1 hour.

Place fluorosilicone in an oven at 115 to 120°C for 10 minutes, remove and leave at room temperature for 10 minutes. Repeat this process a further 3 times.

The above examples were repeated using polyurethane film.

### EXAMPLE 9 : CONTROLLED RELEASE

A 50µm aspirin layer was incorporated into a fluorosilicone polymer using the methodology as described in Examples 1 to 3. The cured composite was subjected to continuous washing with phosphate buffered saline. After 5 months of washing, the antiplatelet effect of aspirin was still evident (aspirin was still being released in a controlled fashion from the composite).

### EXAMPLES 10 AND 11 : COMPARISON OF SMC GROWTH INHIBITION AFTER EXPOSURE TO DEX, ASPIRIN AND GTN

### Example 10

The Human Aortic Vascular SMC cell line was investigated. 96 well plates were seeded and were incubated for at least one day. Medium from the plate was removed and replaced with medium containing varying concentrations of drugs, the drugs being dex, aspirin, GTN or a combination of these drugs. The effects of the drugs were analysed after 72 hours with two viability assays: MTT assay and Neutral Red assay. Absorbance values were read from a microplate reader.

MTT is a yellow tetrazolium salt taken up into cells. In viable cells, the salt was reduced in cytosol or mitochondria to form blue formazan salt. The formazan product was very insoluble and was disolved in dimethylsulphoxide before its absorbance was calculated. In non-viable cells, there was no reaction and the cells remained yellow.

The Neutral Red assay involved adding neutral red solution, a red dye to cells. In viable cells, the dye stained lysosomes. The cells were incubated with neutral red solution for three hours (to enable dye to penetrate cells), a neutral red destain solution was then added to remove the dye. The intensity of the red colour equated to the number of viable cells.

Sterile coverslips were placed into the wells of a 24 well plate. FFCs (bone cells) were seeded into the wells at a seeding density of 5x10³ cells. The key component of the extacellular framework of arterial media was collagen. Collagen production was investigated with specific antibodies labelled with fluorescent tags. The samples were examined under the Confocal Laser Scanning Microscope (CLSH). Cells have endogenous biotin. If the cells have made collagen, fibre networks surround the cells. Once cells were confluent, avidin was added to block the endogenous biotin. Avidin and biotin have a very high affinity to each other. Cells were fixed with formalin to stop further metabolism. Casein was added to block non-specific binding of the protein. (Proteins are naturally 'sticky' and bind to coverslips and plastic. By binding to casein, antibodies were prevented from binding to these areas). Goat anti collagen-biotin antibody (primary antibody) was added to positive control wells and specifically bound to the collagen produced. Finally, the avidin-FITC (secondary antibody) was added to the positive and negative control wells. Neither the primary nor secondary antibodies were added to the control control wells. Isothiocyanate (FITC) binds to the biotin that was on the antibody. FITC fluoresces and so the bound FITC was identifiable. Under the CLSH, there was fluorescence on the positive control coverslips if collagen was produced and some degree of auto-fluorescence on the negative and control coverslips due to non-specific binding.

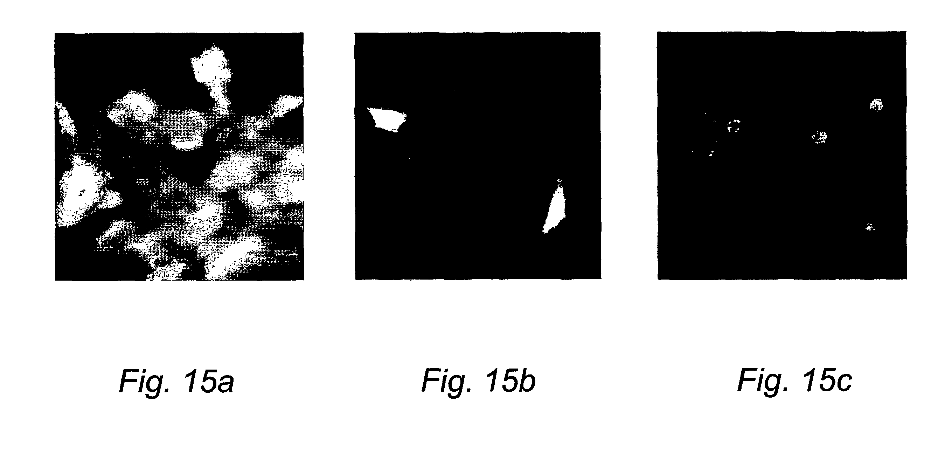

There was still some bright fluorescence in the negative control when it should have been similar to the control results (see Fig. 15). It was thus suspected that there was still some degree of non specific binding. The experiment was repeated using an alternative blocking agent bovine serum albumin (BSA) instead of casein).

### Example 11

The method of Example 10 was repeated using BSA as a blocking agent rather than casein.

HA-VSCMs were cultured on silicone based film membranes supplied by Vascutek. 2 membrane types were tested: uncoated FISi membrane and FISi membrane coated with GTN. Cells were seeded at 5x10³ seeding density. Cells were cultured for a week and the medium was changed every two days. It was found that it was difficult to ensure the cells adhered to the films due to the hydrophobicity of the films meaning that the films floated on the medium. This problem was overcome by sticking the coverslips to the plate with double sided tape before the wells were seeded. Furthermore the cells were washed off during staining or the cells were saved for too long. Some cells were saved by introducing a gentle washing step. On the seventh day, the coverslips were stained and viewed under the CLSM for viability studies.

The following were used as viability stains; CFDA (carboxyfluorescein diacetate) is a vital stain that penetrates cell membranes. Viable cells contain esterase enzymes that remove the diacetate from the molecule thus forming CF which is fluorescent. Once CF if formed, it accumulates in the membrane and viable cells with fluoresce bright green.

Ethidium Bromide: is a vital dye that is only able to pass through the compromised membrane of dead cells and stain them red.

Acridine Orange: is a dye which intercalates with DNA and RNA by intercalation or electrostatic attraction. This cationic dye has green fluorescence when bound to DNA and red when bound to RNA.

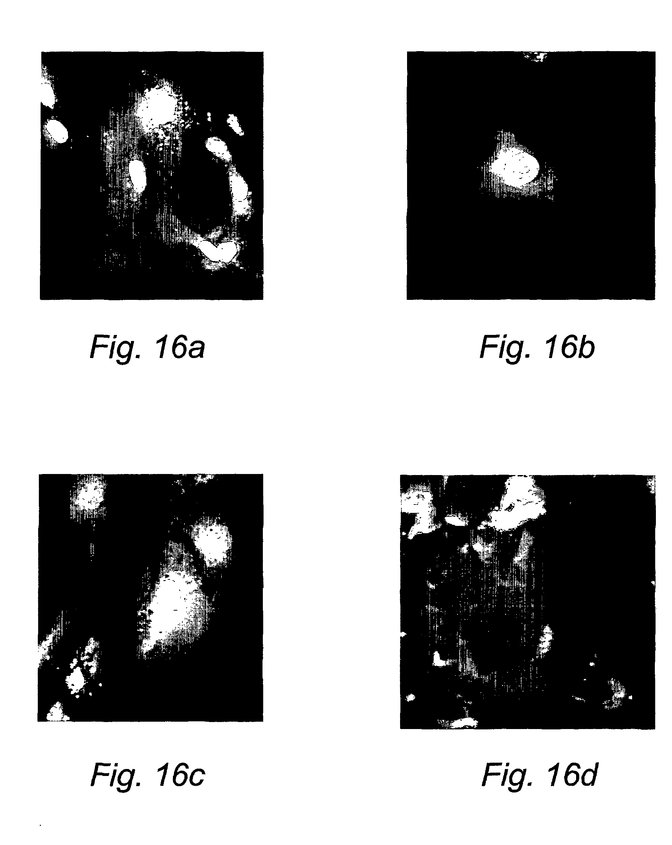

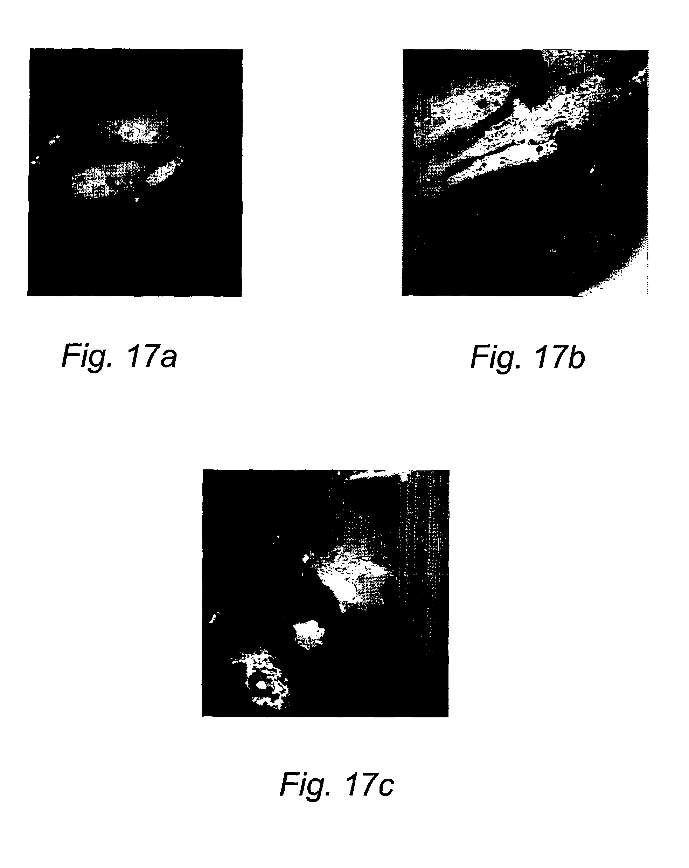

Cells grew better in glass than plastic coverslips (see Fig. 16a and b). Morphologically, cells looked healthy in both controls. In the FISi + GTN membranes, cells grew fairly well (see Fig. 16c). Only dead cells and cell remnants were visible in the FISi only membranes (see Fig. 16d). On the GTN + FISi membrane, there was intense red staining in cytoplasm and green elsewhere. Cells preferred to grow on the uncoated edge of the membranes and became sparse and contracted on the membranes (see Fig. 17).

High doses of dex and aspirin appear to have a beneficial role in the treatment of vascular proliferative disorder. GTN appears to promote VSM relaxation and may be considered as an endothelium interactive vasodilator. GTN does not significantly decrease cell growth compared to FISi only film membranes however.

## Claims

1. A method of producing a biocompatible composite, said method comprising the step of combining a polymer and an active ingredient in solution form, wherein ultrasound having a frequency of 20 to 90 kHz is used in an ultrasonication step during the combination of said polymer and said active ingredient.

2. A method according to Claim 1 wherein the polymer/active ingredient combination is exposed to ultrasound for a time sufficient to achieve an even distribution of active ingredient throughout the composite.

3. A method as claimed in either one of Claims 1 and 2 wherein the polymer is cured prior to exposure to the active ingredient and the ultrasonication step.

4. A method as claimed in either one of Claims 1 and 2 wherein the ultrasonication step is prior to curing of the polymer.

5. A method as claimed in any preceding Claim wherein the active ingredient and polymer are subjected to ultrasound prior to being combined, and after being combined.

6. A method as claimed in any preceding Claim wherein the active ingredient is poorly soluble in water.

7. A method as claimed in any preceding claim wherein the active ingredient is aspirin, an antibiotic, polypeptide, steroidal hormone, anti-platelete agent, anti-proliferative agent, anti-microbial agent, NO donor or a combination thereof.

8. A method as claimed in Claim 7 wherein the active ingredient is aspirin, dexamethasaone or glycerol trinitrate.

9. A method as claimed in any preceding claim wherein the polymer is a silicone, silicone-based polymer, polyester, polyurethane, polyorthoester, poly-α-hydroxy acid, co-polymers thereof or any combination thereof.

10. A method as claimed in any preceding Claim wherein the polymer is a fluorosilicone, polyglycolide (PgA), polylactide (PLA), polyhydroxybutyrate (PHB), or copolymers thereof.

11. A method as claimed in any preceding Claim wherein the frequency of ultrasound used is 40 to 90 KHz.

12. A composite obtainable from the method as claimed in any one of Claims 1 to 11 comprising a polymer and an active ingredient, said active ingredient being evenly dispersed in said polymer.

13. A composite as claimed in Claim 12 wherein the active ingredient is aspirin.

14. A vascular graft comprising a composite as claimed in either one of Claims 12 and 13.

## Patentansprüche

1. Ein Verfahren zum Herstellen einer biokompatiblen Zusammensetzung, wobei das Verfahren den Schritt des Verbindens eines Polymers und eines Wirkstoffs in Lösungsform beinhaltet, wobei Ultraschall mit einer Frequenz von 20 bis 90 kHz in einem Ultraschallschritt während des Verbindens des Polymers und des Wirkstoffs verwendet wird.

2. Verfahren gemäß Anspruch 1, wobei die Polymer/Wirkstoff-Verbindung für eine Zeit lang Ultraschall ausgesetzt wird, die ausreichend ist, um eine gleichmäßige Verteilung von Wirkstoff in der ganzen Zusammensetzung zu erzielen.

3. Verfahren gemäß Anspruch 1 oder 2, wobei das Polymer vulkanisiert wird, bevor es dem Wirkstoff und dem Ultraschallschritt ausgesetzt wird.

4. Verfahren gemäß Anspruch 1 oder 2, wobei der Ultraschallschritt vor dem Vulkanisieren des Polymers stattfindet.

5. Verfahren gemäß einem der vorhergehenden Ansprüche, wobei der Wirkstoff und das Polymer vor dem Verbinden und nach dem Verbinden Ultraschall unterzogen werden.

6. Verfahren gemäß einem der vorhergehenden Ansprüche, wobei der Wirkstoff in Wasser schlecht löslich ist.

7. Verfahren gemäß einem der vorhergehenden Ansprüche, wobei der Wirkstoff Aspirin, ein Antibiotikum, Polypeptid, Steroidhormon, Antithrombozyten-Mittel, antiproliferatives Mittel, antimikrobielles Mittel, KEIN Donator oder eine Verbindung davon ist.

8. Verfahren gemäß Anspruch 7, wobei der Wirkstoff Aspirin, Dexamethason oder Trinitroglycerin ist.

9. Verfahren gemäß einem der vorhergehenden Ansprüche, wobei das Polymer ein Silikon, ein auf Silikon basierendes Polymer, Polyester, Polyurethan, Polyorthoester, Poly-α-Hydroxysäure, Copolymere davon oder jede beliebige Verbindung davon ist.

10. Verfahren gemäß einem der vorhergehenden Ansprüche, wobei das Polymer ein Fluorsilikon, Polyglycolid (PgA), Polylactid (PLA), Polyhydroxybutyrat (PHB) oder Copolymere davon ist.

11. Verfahren gemäß einem der vorhergehenden Ansprüche, wobei die Frequenz des verwendeten Ultraschalls 40 bis 90 kHz beträgt.

12. Eine Zusammensetzung, die aus dem Verfahren gemäß einem der Ansprüche 1 bis 11 erhalten werden kann, die ein Polymer und einen Wirkstoff beinhaltet, wobei der Wirkstoff in dem Polymer gleichmäßig dispergiert ist.

13. Zusammensetzung gemäß Anspruch 12, wobei der Wirkstoff Aspirin ist.

14. Ein Gefäßtransplantat, das eine Zusammensetzung gemäß Anspruch 12 oder 13 beinhaltet.

## Revendications

1. Une méthode de production d'un composite biocompatible, ladite méthode comportant l'étape de combiner un polymère et un principe actif sous forme de solution, dans laquelle des ultrasons ayant une fréquence allant de 20 à 90 kHz sont utilisés lors d'une étape d'ultrasonication pendant la combinaison dudit polymère et dudit principe actif.

2. Une méthode selon la revendication 1 dans laquelle la combinaison polymère/principe actif est exposée à des ultrasons pendant une durée suffisante pour parvenir à une répartition uniforme du principe actif dans le composite tout entier.

3. Une méthode telle que revendiquée dans l'une ou l'autre des revendications 1 et 2 dans laquelle le polymère est durci avant d'être exposé au principe actif et à l'étape d'ultrasonication.

4. Une méthode telle que revendiquée dans l'une ou l'autre des revendications 1 et 2 dans laquelle l'étape d'ultrasonication précède le durcissement du polymère.

5. Une méthode telle que revendiquée dans n'importe quelle revendication précédente dans laquelle le principe actif et le polymère sont soumis à des ultrasons avant d'être combinés, et après avoir été combinés.

6. Une méthode telle que revendiquée dans n'importe quelle revendication précédente dans laquelle le principe actif se dissout mal dans l'eau.

7. Une méthode telle que revendiquée dans n'importe quelle revendication précédente dans laquelle le principe actif est de l'aspirine, un antibiotique, un polypeptide, une hormone stéroïde, un agent antiplaquettaire, un agent antiproliférant, un agent antimicrobien, un donneur de NO ou une combinaison de ceux-ci.

8. Une méthode telle que revendiquée dans la revendication 7 dans laquelle le principe actif est de l'aspirine, de la dexaméthasone ou du trinitrate de glycérol.

9. Une méthode telle que revendiquée dans n'importe quelle revendication précédente dans laquelle le polymère est une silicone, un polymère à base de silicone, un polyester, un polyuréthane, un polyorthoester, un acide poly-α-hydroxy, des copolymères de ceux-ci ou toute combinaison de ceux-ci.

10. Une méthode telle que revendiquée dans n'importe quelle revendication précédente dans laquelle le polymère est un fluorosilicone, un polyglycolide (PgA), un polylactide (PLA), un polyhydroxybutyrate (PHB), ou des copolymères de ceux-ci.

11. Une méthode telle que revendiquée dans n'importe quelle revendication précédente dans laquelle la fréquence des ultrasons utilisés va de 40 à 90 kHz.

12. Un composite pouvant être obtenu d'après la méthode telle que revendiquée dans n'importe laquelle des revendications 1 à 11 comportant un polymère et un principe actif, ledit principe actif étant dispersé de façon uniforme dans ledit polymère.

13. Un composite tel que revendiqué dans la revendication 12 dans lequel le principe actif est de l'aspirine.

14. Un greffon vasculaire comportant un composite tel que revendiqué dans l'une ou l'autre des revendications 12 et 13.
